# EUROPEAN PATENT APPLICATION

(11) **EP 4 582 811 A1**
(43) Date of publication of application: **09.07.2025**
(21) Application number: 23859325.5
(22) Date of filing: 29.08.2023
(51) Int. Cl.: G01N 33/569, G01N 33/58, G01N 33/577, G01N 33/558, G01N 33/543, G01N 1/10, G01N 1/28, A61B 10/00

(54) **DETECTION APPARATUS**

(30) Priority: 29.08.2022 CN 202211065608; 07.09.2022 CN 202211091536; 25.10.2022 CN 202211314003
(71) Applicant: Leadway (HK) Limited, Sheung Wan, Hong Kong (CN)
(72) Inventor: CAI, Zhengjun, Hangzhou, Zhejiang 310030 (CN); SHANG, Tao, Hangzhou, Zhejiang 310030 (CN); TANG, Linyong, Hangzhou, Zhejiang 310030 (CN)
(74) Representative: Schiweck Weinzierl Koch Patentanwälte Partnerschaft mbB
(86) International application number: PCT/CN2023/115406
(87) International publication number: WO 2024/046295

(57) **Abstract**

The present invention provides a test device, including a sample collector (200) and a test kit (300), the test kit (300) uses to store a test strip (100). The sample collector (200) includes a sampling head (203), a handle (201) and a connecting portion (202) between the sampling head (203) and the handle (201); and the test kit (300) includes a sample addition hole (3) and a buckle (10). When the sample collector (200) is placed into the test kit (300), the connecting portion (202) of the sample collector (200) is fastened to the buckle (10) of the test kit (300), and the connecting portion (202) of the sample collector (200) includes a wedge-shaped structure (202a) wide at the top and narrow at the bottom, the wedge-shaped structure (202a) being located at the point (202) where the sample collector (200) is in fastening fit with the buckle (10). By using the wedge-shaped structure (202a) of the connecting portion (202) of the sample collector (200) of the present invention, it is easier for the connecting portion (202a) to pass through the buckle (10) of the test kit (300), thereby reducing the squeezing force applied to the sample collector (200), and then reducing the impact of the sample collector (200) on the squeezed liquid sample, thereby effectively reducing the splashing of the liquid sample and avoiding contaminating the operator and the environment by the splashed sample.

## Description

### Field of the Invention

The present invention relates to the technical field of rapid medical testing, in particular to a portable test device and a sample collector.

### Background of the Invention

The carrier of traditional disposable point-of-care testing products is basically in the form of a test strip or a test kit, for example, the test strip of a test product in the lateral flow form generally includes a bottom card, a sample pad, a label binding pad (which may also be referred to as a label pad, and generally uses glass fiber as a carrier), a test pad (which generally uses an NC film as a carrier) and an absorbent pad (which generally uses an absorbent material such as filter paper) are adhered to the bottom card in a manner of being superposed on each other in sequence from upstream to downstream, and the immunochromatographic principle is used for delivering the sample on the test strip and obtaining test results. The label pad includes labels that can be bound to an analyte, for example, latex, colloidal gold, fluorescent microspheres, etc labeled with antigens or antibodies. The test pad is generally provided with a test line and a control line. As the sample flows on the test strip, the label will be captured and gathered or not captured on the test line. The presence/absence or concentration of the analyte is determined according to the signal of the label, such as a color signal or a fluorescence signal. The control line can be used to determine whether the test strip is valid or to position an instrument when the test result is being read. As for the test kit, the test strip is placed between an upper cover and a lower plate, the upper cover is provided with a sample addition hole correspondingly above the sample pad of the test strip, and the upper cover is provided with an observation window correspondingly above the test pad of the test strip.

Disposable point-of-care testing and diagnostic products for the testing of diseases or other physical conditions using body fluids such as urine, blood or other tissue fluids of a human body have been commonly used all over the world, and they can be applied in laboratories operated by professionals, or places such as home, schools, shopping malls, road checkpoints, and customs operated by non-professionals themselves.

Such traditional test products are merely simple test strips or test kits, and when sampling with a sampling stick for testing, it is necessary to put the sampling stick into a collection bottle after completion of the sampling, the sample on the sampling stick is squeezed into the collection bottle and then is dropwise added to the sample pad or the sample addition hole, as shown in Figs. 7 and 8 of U.S. Patent Application US20040237674A1, but such sample addition operation is relatively cumbersome. Chinese Patent CN200420110153.3 and Chinese Patent CN201010164579.7 improve the test kit, respectively, and simplify the steps of the sample addition operation. The sample addition hole of the test kit in Chinese Patent CN200420110153.3 protrudes out of the upper cover of the test kit to form a receiving chamber, and the internal space of the receiving chamber can receive the absorbent material on the sampling stick therein, and the liquid sample is squeezed onto the sample pad by squeezing. The sample addition hole in Chinese patent CN201010164579.7 is provided with a channel, which allows passage of a sampling head, the liquid sample is squeezed out by squeezing and the sample is delivered to the sample pad via a drainage member. The two Chinese patents have some problems below, for example, the user needs to press on the sampling stick all the time to maintain the squeezing state until the liquid sample is completely squeezed, and if the sampling stick is released in the midway of the process, the liquid sample retained in the sampling stick after the loss of the squeezing force on the sampling stick will not be able to reach the sample pad, which increases the risk of insufficient sample volume; it is also possible that the sampling stick after being released falls out from the sample addition hole or the sample addition channel to contaminate the ambient environment; and the sample addition hole or the sample addition channel formed in the upper cover is blocked by the sampling stick during sample addition, the liquid sample cannot be delivered to the test strip below in time so as to be transported away but is accumulated in the sample addition region, and the squeezed sample may be squeezed by the sampling stick to spill or splash out of the sample addition region, and even splash onto the body of an operator, causing danger to the operator. In addition, the existing sampling stick needs to exert a larger acting force when squeezing a sampling component, and the route of the sampling stick is also larger, so it is likely to cause a larger impact on the squeezed liquid sample, and the squeezed sample may be squeezed by the sampling stick to spill or splash out of the sample addition region, and even splash onto the body of an operator, causing danger to the operator.

Therefore, how to provide a test device capable of achieving point-of-care testing and also preventing the sample from splashing out of the sample addition area in the sample addition process, is a technical problem urgently to be solved by the person skilled in the art at present.

### Summary of the Invention

The present invention provides a test device, comprising a sample collector and a test kit. An objective of the present invention is to solve the technical problem that the liquid sample collected by the sample collector is not likely to be squeezed out sufficiently, and to prevent the liquid sample from splashing in the squeezing process.

In order to achieve this objective, the present invention provides the following technical solution.

The present invention provides a test device, comprising a sample collector and a test kit, the test kit is used to store a test strip. The sample collector includes a sampling head, a handle and a connecting portion between the sampling head and the handle; and the test kit comprises a sample addition hole and a buckle. When the sample collector is placed into the test kit, the connecting portion of the sample collector is fastened to the buckle of the test kit, and the connecting portion of the sample collector comprises a wedge-shaped structure wide at the top and narrow at the bottom, the wedge-shaped structure being located at the point where the sample collector is in fastening fit with the buckle.

More specifically, the test device, comprises a sample collector, and a test kit in which a test strip is disposed; the sample collector comprises a sampling head, a handle and a connecting portion between the sampling head and the handle; the test kit comprises a sample addition hole, an enclosure and a buckle, the enclosure enclosing the sample addition hole therein and forming a storage chamber, and the connecting portion of the sample collector comprising a wedge-shaped structure wide at the top and narrow at the bottom; the buckle of the test kit comprises a pair of inverted clamping hooks, the hook tips of which face inward towards each other, and after the sampling head of the sample collector is placed into the storage chamber of the test kit, the wedge-shaped structure of the sample collector squeezes the buckle downward, causing the pair of inverted clamping hooks to open outward; and after the wedge-shaped structure passes through the hook tips, the clamping hooks spring back and hook the wedge-shaped structure, and the sample squeezed out of the sampling head enters the test strip through the sample addition hole.

Further, the wedge-shaped structure comprises at least one beveled or cambered surface which guides the clamping hooks to open outward when the wedge-shaped structure squeezes the buckle downward.

Further, the shape of at least the lower part of the cross section of the wedge-shaped structure comprises: an inverted trapezoid, an inverted triangle, a combination of an inverted trapezoid and a rectangle, a semicircle, or a shape approximate to a semicircle. Further, the cross section of the wedge-shaped structure is in the shape of inverted " 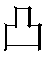 ". Further, the width of the bottom of the wedge-shaped structure is less than the distance between the two hook tips of the clamping hook.

Further, the width of the bottom of the wedge-shaped structure is less than one half of the width of the top thereof.

Further, the test kit further comprises a second buckle that catches the sampling head of the sample collector after the sampling head of the sample collector is placed into the storage chamber of the test device.

Further, the second buckle is disposed in the storage chamber of the test device.

Further, the tail end of the second buckle is provided with a bump, and the sampling head of the sample collector is provided with a recess, so that when the sampling head of the sample collector is placed into the storage chamber of the test kit, the bump of the second buckle is buckled in the recess of the sampling head of the sample collector. The present invention further provides a sample collector, comprising a sampling head, a handle and a connecting portion between the sampling head and the handle, the connecting portion of the sample collector comprising a wedge-shaped structure wide at the top and narrow at the bottom.

Further, the wedge-shaped structure comprises at least one beveled or cambered surface. Further, the shape of at least the lower part of the cross section of the wedge-shaped structure comprises: an inverted trapezoid, an inverted triangle, a combination of an inverted trapezoid and a rectangle, a semicircle, or a shape approximate to a semicircle. Further, the cross section of the wedge-shaped structure is in the shape of inverted " 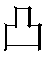 ". Further, the width of the bottom of the wedge-shaped structure is less than one half of the width of the top thereof.

### Beneficial effects

The test device described in the present invention is provided with a wedge-shaped structure wide at the top and narrow at the bottom at the connecting portion of the sample collector thereof, and preferably, the wedge-shaped structure further comprises a pair of beveled or cambered surfaces having a guiding function, so that it is easier for the wedge-shaped structure to pass through the pair of clamping hooks on the test kit, thereby reducing the squeezing force applied to the sample collector. In addition, the size of the bottom of the wedge-shaped structure is smaller than the distance between the hook tips of the pair of clamping hooks in cooperation with the wedge-shaped structure on the test kit, so that the bottom of the wedge-shaped structure enters the underside of the hook tips of the pair of clamping hooks without being blocked, and thus shortens the sliding distance of the wedge-shaped structure along the clamping hooks on the test kit during the squeezing process, which in turn effectively reduces the impact of the sample collector on the squeezed liquid sample, thereby effectively reducing the splashing of the liquid sample. Therefore, the present invention can effectively reduce the splashing of the liquid sample out of the test device in the testing process, prevent the liquid sample from contaminating the environment around the testing, prevent the liquid sample from splashing onto the body of an operator, and reduce the risk of physical harm to the operator. Moreover, the test device is clamped with the wedge-shaped structure by means of clamping hooks, so that the sample collector is kept on the test kit, the sampling pad can be squeezed sufficiently, and the sample is fully squeezed out, which avoids the problem that insufficient added sample volume leads to a failed test, and avoids contamination of the ambient environment resulted from the fact that the sampling stick falls out of the sample addition hole or the sample addition channel.

### Brief Description of the Drawings

Fig. 1 is a schematic diagram of the initial state of use of a test device of the present invention, i.e., a schematic diagram at the point that a sample collector is just inserted into a test kit.
Fig. 2 is a schematic diagram showing the point that the sample collector of the test device of the present invention is completely inserted into the test kit.
Fig. 3 is a stereoscopic exploded schematic diagram of the test device of the present invention.
Fig. 4 is a schematic diagram of a test strip in a lateral flow form.
Fig. 5 is a stereoscopic schematic diagram of a sample collector.
Fig. 6 is stereoscopic exploded schematic diagram of the assembled test kit of the present invention.
Fig. 7 is a top view of the test kit in Fig. 6.
Fig. 8 is a sectional view of Fig. 7 in the direction of A-A.
Fig. 9 is a sectional view of Fig. 7 in the direction of B-B.
Fig. 10 is a top view of Fig. 2.
Fig. 11 is a partially enlarged schematic diagram within circle "A" in Fig. 5.
Fig. 12 is a schematic diagram of the start of inserting the sample collector into the test kit.
Fig. 13 is a sectional diagram of the process of inserting the sample collector into the test kit.
Fig. 14 is a sectional diagram at the point that the sample collector has been completely inserted into the test kit.
Fig. 15 is a partially enlarged schematic diagram within circle "B" in Fig. 14.
Fig. 16 is a sectional view of Fig. 11 in the direction of C-C.
Fig. 17 to Fig. 23 are schematic diagrams of an altered design of Fig. 16.
Figs. 24 to 26 are diagrams presenting the process of inserting the connecting portion into the buckle according to the design solutions shown in Fig. 23.
Figs. 27 to 29 are diagrams presenting the process of inserting the connecting portion into the buckle according to the design solutions shown in Fig. 16.
Fig. 30 is a partially enlarged schematic diagram within circle "C" in Fig. 24.
Fig. 31 is a partially enlarged schematic diagram within circle "D" in Fig. 27.
Fig. 32 is a comparative schematic diagram of the two solutions shown in Fig. 24 and

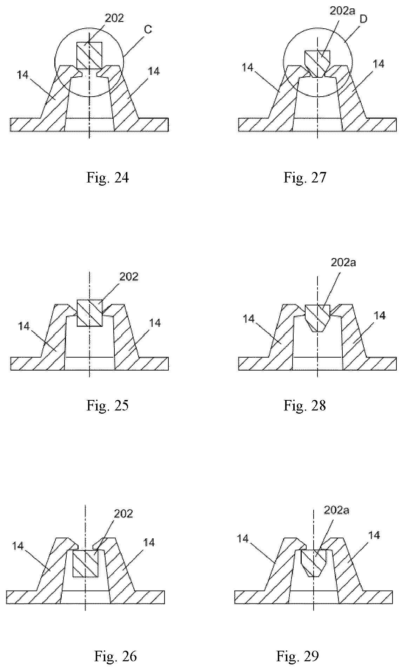

Fig. 27 after they are overlapped together (the dotted line is the solution of Fig. 24 and the solid line is the solution of Fig. 27).

### Detailed Description of the Embodiments

The "lower part" in the specification of the present patent includes the "bottom" of a component and a part extending upwards from the "bottom" of the component by an appropriate distance.

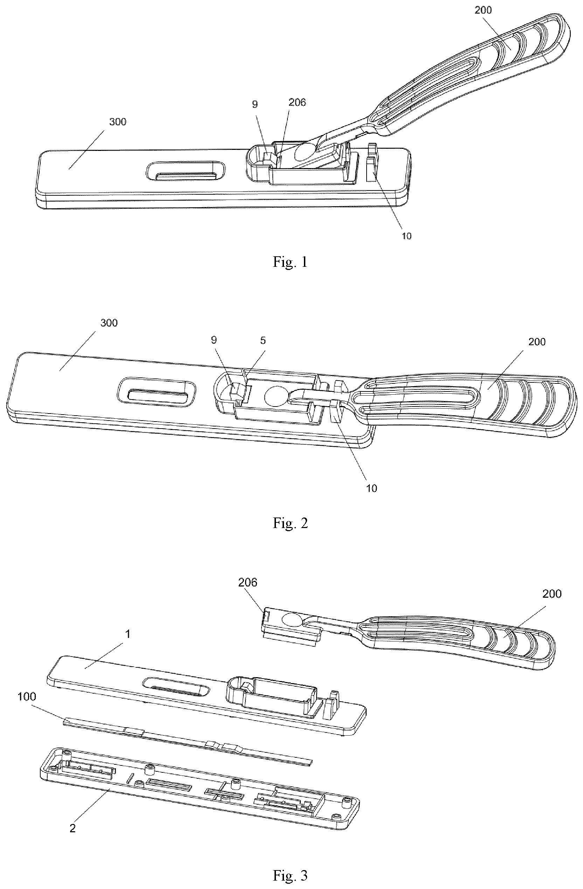

A test device, as shown in Figs. 1 to 9, includes a test kit 300 and a sample collector 200 used in cooperation with the test kit. The test kit includes an upper cover 1, a lower plate 2, and a test strip 100 located between the upper cover and the lower plate of the test kit. In one example, the upper cover 1 and the lower plate 2 are buckled to each other to install the test strip 100 within the test kit 300.

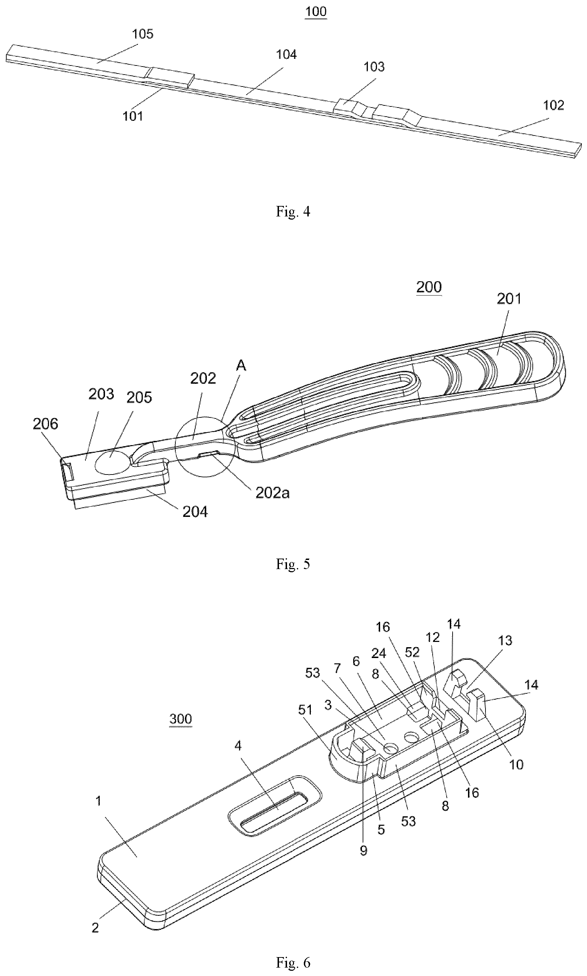

As shown in Fig. 4, the test strip 100 is a test strip in the lateral flow form, which includes a bottom card 101, and a sample pad 102, a label binding pad 103, a test pad 104 and an absorbent pad 105 are adhered to the bottom card 101 in a manner of being superposed on each other in sequence from upstream to downstream (i.e., the transfer direction of the liquid sample). The test strip 100 may also be a test strip in a vertical flow form or a test strip in other forms which is known to the person skilled in the art.

The sample collector 200 as shown in Fig. 5 includes a handle 201, a connecting portion 202, and a sampling head 203, the connecting portion 202 being between the handle 201 and the sampling head 203. The sampling head 203 includes a sampling pad 204 fixed to the sampling head 203 in a manner such as buckling or adhering. The sampling pad 204 may employ an absorbent tampon, a sponge, an absorbent fiber or a porous polymer material or the like, or other absorbent material well known in the art, such as polyvinyl alcohol. The sampling head 203 may also be provided with a transparent indication region 205 located on the sampling pad 204, an indicator test strip is stored in the indication region 205, so that when the sampling pad 204 absorbs a sufficient liquid sample, the liquid sample will come into contact with the indicator test strip, causing the indicator test strip to change from a first color to a second color, thus an operator can determine whether a sufficient sample is collected in accordance with the change in color of the indicator test strip. The handle 201 may be designed as a flat configuration with a certain curvature to facilitate holding by the operator, and the connecting portion 202 is designed in a thin-neck shape.

As shown in Fig. 6, the upper cover 1 of the test kit 300 is provided with a sample addition hole 3 and an observation window 4, the sample addition hole 3 is located above the sample pad 102 of the test strip, and the observation window 4 is located above the test pad 104 of the test strip. An enclosure 5 is disposed in the region around the sample addition hole 3. The enclosure 5 may enclose the sample addition hole 3 therein in a manner of standing on the upper surface of the upper cover and form a storage chamber 6 therein. The enclosure 5 may also enclose the sample addition hole 3 therein in a sunken manner in the way of extending downwards from the upper surface of the upper cover towards the interior of the test kit and form the storage chamber 6. The enclosure 5 may also have a part standing on the upper surface of the upper cover 1 and have another part enclosing the sample addition hole 3 therein in a manner of extending towards the interior of the test kit and form the storage chamber 6. The enclosure 5 is substantially in a wall structure, in which a storage chamber 6 is located. The enclosure 5 includes a front enclosure 51, a rear enclosure 52, and two side enclosures 53. When the sampling head 203 of the sample collector 200 is inserted into the storage chamber 6, the front enclosure 51 is located in the direction of the top end of the sample collector, and the rear enclosure 52 is located in the direction of the connecting portion 202 of the sample collector. The region within the storage chamber 6 located in the vicinity of the front enclosure 51 can be referred to as the front end of the storage chamber, and the region within the storage chamber located in the vicinity of the rear fence 52 can be referred to as the rear end of the storage chamber 6.

The storage chamber 6 includes a bottom plate 7 at the bottom, and the sample addition hole 3 is formed in the bottom plate 7. After the sampling head 203 is completely inserted into the storage chamber 6, the bottom of the sampling pad 204 presses against the bottom plate 7, the sampling head 203 squeezes the sampling pad 204 downwards so as to squeeze the liquid sample out of the sampling pad 204. The squeezed liquid sample enters to the sample pad 102 of the test strip through the sample addition hole 3, and then the testing is started. **In** an example, the bottom plate 7 may be a part of the top plate of the upper cover, while the enclosure 5 is enclosed by the top plate of the upper cover projecting upwards at an appropriate height to form a circle, and the cavity within the circle forms the storage chamber 6. The bottom plate 7 may be provided with a plurality of sample addition holes 3, for example, two sample addition holes spaced apart, above the sample pad 102 of the test strip, so that on the one hand, the squeezed sample can be introduced onto the sample pad 102 of the test strip faster, and on the other hand, the strength of the bottom plate 7 is maintained so that the squeezing plate is not prone to deformation in the squeezing process.

**In** one example, three diversion grooves 8 are provided at the joint of the bottom plate 7 and the rear enclosure 52, the diversion grooves 8 penetrate through the bottom plate 7, therefore, the diversion grooves 8 are in communication with the interior of the test kit, so that the squeezed liquid sample can be introduced into the test kit or to the sample pad 102 of the test strip, and is absorbed by the sample pad 102 of the test strip.

**In** another example, the lower part of the rear enclosure 52 of the storage chamber 6 is provided with drain holes 16, the drain holes 16 penetrate through the bottom plate 7, and the drain holes 16 are in communication with the interior of the test kit, so that the liquid sample flowing to the rear enclosure 52 can enter the interior of the test kit 300 through the drain holes 16.

In an another example, the diversion groove 8 in the bottom plate corresponds to the drain hole 16 on the lower edge of the rear enclosure 52, and the diversion groove 8 extends all the way to the drain hole 16 at the lower part of the rear enclosure. More specifically, the diversion groove 8 located in the bottom plate and the drain hole 16 located in the rear enclosure are connected to form a right-angled opening or an "L"-shaped opening. Such a design may further increase the flux of the liquid sample flowing into the test kit, avoiding that more liquid sample is retained in the storage chamber 6 in the process of squeezing the sample collector, which causes the liquid sample to splash out of the storage chamber 6.

In an another example, at the rear end of the storage chamber 6 of the test kit, the bottom plate 7 is provided with diversion grooves 8, the lower part of the rear enclosure 52 is provided with drain holes 16, the lower part of the side enclosure 53 is provided with a side drain hole 24, the diversion groove 8 extends towards the drain hole 16 and the side drain hole 24, respectively, and intersects the drain hole 16 and the side drain hole 24, and the drain hole 16 and the side drain hole 24 are connected, so that at each of the two corners of the rear of the storage chamber 6, an opening consisting of the diversion groove 8, the drain hole 16 and the side drain hole 24 is formed to divert the liquid sample gathered at the rear of the storage chamber 6.

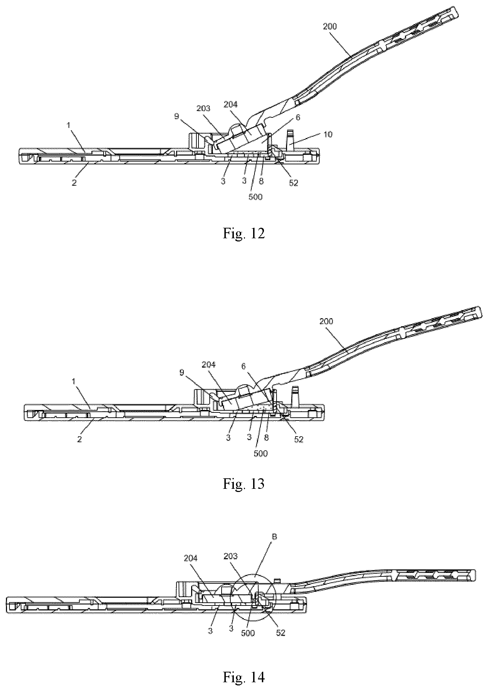

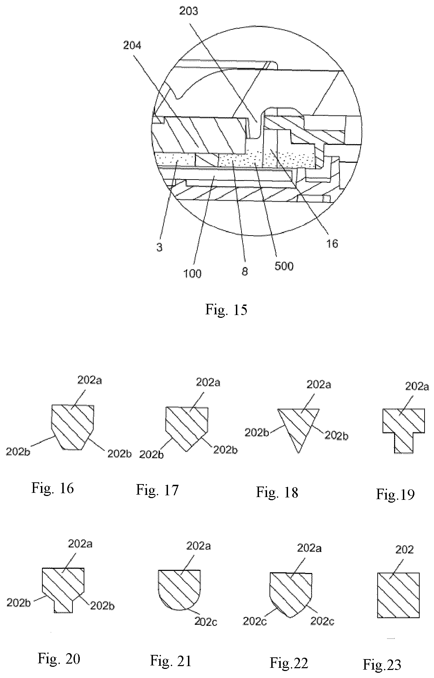

Figs. 12, 13 and 14 illustrate the process of inserting the sample collector 200 into the test kit 300. Fig. 15 illustrates a schematic diagram of the distribution of the squeezed liquid sample 500 (shown by the densely distributed dots in Fig. 15) after the sample collector 200 is completely inserted into the test kit 300.

The opening size and height of the drain hole 16, the distance between the drain hole 16 and the bottom plate 7 (for example, the drain hole may penetrate through the bottom plate or may be in the bottom plate without penetrating through the bottom plate), and the number of the drain hole, etc. may be adjusted according to the sampling quantity of the sample collector, the capacity of the storage chamber, the sample injection rate of the sample addition hole and other factors.

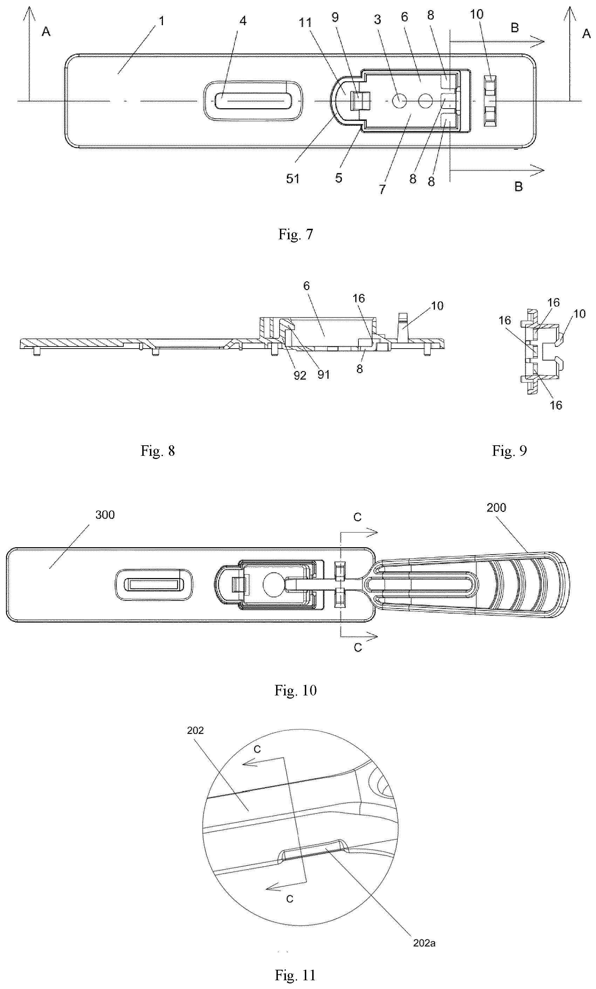

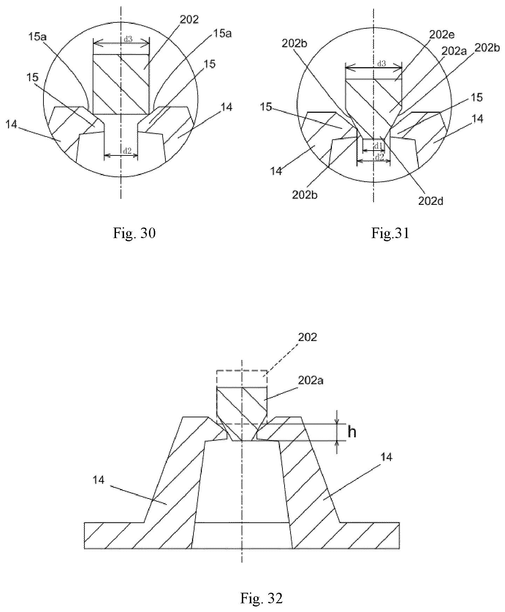

As shown in Figs. 1 and 2, the upper cover 1 is also provided with a fastening device for the sample collector. Preferably, the fastening device and the upper cover 1 are integrally formed through plastics. The fastening device includes a buckle 10. Preferably, the buckle 10 is formed on the top plate of the upper cover 1 and is located near or beside the storage chamber 6; and more preferably, the buckle 10 is located near or beside the rear enclosure 52. The buckle 10 may also be located in other suitable position as long as it is capable of implementing the function of the present invention, and therefore the position of the buckle 10 should not limit the scope of the technical solution claimed by the present invention. In a preferred example, the buckle 10 includes a pair of inverted clamping hooks 14 (the clamping hooks 14 should have an appropriate elasticity) extending upward at a suitable height from the top plate of the upper cover 1, the hook tips 15 of the pair of clamping hooks (as shown in Fig. 30) facing inward towards each other. Correspondingly, the connecting portion 202 of the sample collector 200 includes a wedge-shaped structure 202a wide at the top and narrow at the bottom (as shown in Fig. 5 and 11). Refer to Figs. 12 to 14 and Figs. 27 to 29, it is shown that after the sampling head 203 of the sample collector is placed in the storage chamber 6 of the test kit, the lower part of the wedge-shaped structure 202a of the sample collector corresponds to the buckle 10 in position. When an appropriate amount of downward pressure is applied to the handle, the wedge-shaped structure 202a squeezes the buckle 10 downwards, and the wedge-shaped structure 202a or the beveled surface 202b or cambered surface 202c of the wedge-shaped structure 202a props up the upper part of the pair of clamping hooks 14, that is, the upper part of the pair of inverted clamping hooks 14 is enabled to generate an appropriate elastic deformation to cause the upper part of the clamping hooks 14 to expand outwards, which in turn causes the gap between the pair of hook tips 15 to become larger, so that the wedge-shaped structure 202a is allowed to pass through the pair of hook tips 15, and then the pair of clamping hooks 14 rebounds and the pair of hook tips 15 hooks the wedge-shaped structure 202a, keeping the sampling head 203 within the storage chamber 6 to continuously squeeze the liquid sample out of the sampling pad 204. Preferably, the top of the pair of inverted clamping hooks 14 is provided with beveled surfaces 15a (as shown in Fig. 30) that guide the wedge-shaped structure 202a to squeeze the hook tips 15, thereby further reducing the resistance to the passage of the wedge-shaped structure 202a through the pair of hook tips 15, that is to say, the acting force applied to the handle 201 of the sample collector is reduced, thus reducing the impact on the squeezed liquid sample, and preventing the liquid sample from splashing out of the storage chamber 6.

As shown in Figs. 16 to 18 and Fig. 20, the wedge-shaped structure 202a includes at least one beveled surface 202b (preferably including a pair of beveled surfaces 202b), or as shown in Figs. 21 and 22, the wedge-shaped structure 202a includes at least one cambered surface 202c (preferably including a pair of cambered surfaces 202c). When the wedge-shaped structure 202a squeezes the clamping hooks 14 downward, the beveled surface 202b or cambered surface 202c guides the upper part of the clamping hooks 14 to expand outwards, i.e., the beveled surface 202b or curved surface 202c squeezes the pair of hook tips 15 or the beveled surfaces 15a of the hook tips outwards to propel the upper part of the clamping hooks 14 to expand outwards, which causes the gap between the pair of hook tips 15 to become larger, so that the wedge-shaped structure 202a is allowed to pass through the pair of hook tips 15 more easily, and then the upper parts of the pair of clamping hooks 14 elastically rebounds and the pair of hook tips 15 hooks the top of wedge-shaped structure 202a.

Figs. 16 to 22 illustrate several specific structures of the wedge-shaped structure 202a, respectively. Overall, the cross section of the wedge-shaped structure 202a generally has a structure with a wide upper part and a narrow lower part, thereby facilitating the insertion of the wedge-shaped structure into the clamping hooks 14. Specifically, in some examples, the shape of the entire cross section of the wedge-shaped structure 202a or the shape of the lower part of the cross section of the wedge-shaped structure includes: an inverted trapezoid or isosceles trapezoid (as shown in Fig. 16), an inverted triangle or isosceles triangle (as shown in Figs. 17 and 18), a combination of an inverted trapezoid and a rectangle (as shown in Fig. 20), a semicircle (as shown in Fig. 21), or a shape approximate to a semicircle (as shown in Fig. 22). In the example shown in Fig. 19, the cross section of the wedge-shaped structure 202a is the cross section of the wedge-shaped structure is in the shape of inverted " 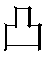 ". In the example shown in Fig. 20, the cross section of the wedge structure 202a may also be considered to be in a shape approximate to the shape of inverted " 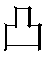 ". In the example shown in Fig. 23, the connecting portion 202 does not include the wedge-shaped structure and has a rectangular cross section, including squares and rectangles.

Figs. 24 to 26 illustrate the process of inserting the connecting portion 202 into the pair of clamping hooks 14 when the connecting portion 202 does not include a wedge-shaped structure, i.e., it has a rectangular cross section (in the example as shown in Fig. 23). Figs. 27 to 29 illustrate the process of inserting the wedge-shaped structure 202a into the pair of clamping hooks 14 when the connecting portion 202 includes the wedge-shaped structure 202a. The two solutions are compared, it is obvious that the solution in which the connecting portion includes the wedge-shaped structure 202a is superior to the solution in which the connecting portion does not include the wedge-shaped structure. This will be analyzed in further details below.

As shown in Fig. 30, when the connecting portion 202 has a rectangular cross section (i.e., the connecting portion 202 does not include the wedge-shaped structure, with the upper and lower parts thereof in the same size), the width size d3 of the cross section must be greater than the distance d2 between the pair of hook tips 15, otherwise, after the connecting portion 202 is under the pair of hook tips 15, the pair of hook tips 15 will not be able to hook the connecting portion 202. As shown in Fig. 31, when the connecting portion 202 includes a wedge-shaped structure 202a, the size d3 of the top of the wedge-shaped structure 202a is larger than the distance d2 between the pair of hook tips 15, and the size d1 of the bottom of the wedge-shaped structure 202a is smaller than the distance d2 between the pair of hook tips 15. Taking the specific structure of the wedge-shaped structure 202a shown in Fig. 16 as an example, when the wedge-shaped structure 202a is squeezed downwards through the pair of hook tips 15, the bottom 202d of the wedge-shaped structure 202a passes through the pair of hook tips 15 at first in a non-squeezed state until a pair of beveled surfaces 202b (or cambered surfaces 202c, as shown in Figs. 21 and 22) of the wedge-shaped structure 202a squeezes the pair of hook tips 15 or the pair of beveled surfaces 15a of the pair of hook tips. Please refer to Fig. 32, it is shown that, compared with the design solution in which the cross section of the connecting portion 202 is rectangular, in the design solution of the wedge-shaped structure, the wedge-shaped structure 202a has descended one more height "h" in the non-squeezed state, i.e., in the process of squeezing the wedge-shaped structure 202a to pass through the pair of hook tips 15, the descending route of the structure is "h" less than that in the design solution that the wedge-shaped structure 202a has a rectangular cross section. In addition, the pair of beveled surfaces 202b or cambered surfaces 202c of the wedge-shaped structure 202a have a guiding effect in this process, greatly reducing the squeezing force that needs to be applied to the handle 201 of the sample collector, so that it is easier for the wedge-shaped structure 202a of the sample collector to enter the interior of the buckle 10, and when the sampling head 203 squeezes out the liquid sample collected by the sampling pad 204, the impact on the squeezed liquid sample is reduced, thereby reducing splashing of the liquid sample and then well overcoming the shortcomings in the prior art and in the technical solution shown Fig. 23.

As shown in Fig. 31, in a preferred solution, the width d1 of the bottom 202d of the wedge-shaped structure is less than one half of the width d3 of the top 202e thereof. The relationship of the width d1 of the bottom 202d of the wedge-shaped structure, the distance d2 between the pair of hook tips 15 when the clamping hooks are not squeezed, and the width d3 of the top 202e of the wedge-shaped structure is that: d1 is less than d2 and d2 is less than d3.

The solution in the present invention that the connecting portion 202 of the sample collector is designed to include the wedge-shaped structure 202a is not only conducive to enabling the connecting portion 202 to squeeze and pass through the pair of hook tips 15 more easily, but also can ensure that the size of the connecting portion 202 in the vertical direction is sufficiently large, and thus ensures that the connecting portion 202 has sufficient strength and rigidity so that the connecting portion 202 is not prone to deformation or fracture in the squeezing process. The pair of clamping hooks 14 is clamped with the wedge-shaped structure 202a to hold the sample collector 200 on the test kit 300, and the sampling pad 204 is sufficiently squeezed to squeeze the sample out sufficiently.

Please refer to Fig. 1, Fig. 2, Figs. 6 to 8, and Figs. 12 to 14, it is shown that the test kit 300 further includes a second buckle 9 that clamps the front end of the sampling head 203 of the sample collector after the sampling head 203 of the sample collector is placed into the storage chamber 6 of the test device. Preferably, the second buckle 9 and the buckle 10 are located at the two ends or on the periphery of the two ends of the sampling head 203 of the sample collector, and such a design is more helpful in applying an appropriate pressure to the sampling head 203, and is more conducive to quickly squeezing the liquid sample collected by the sampling head 203 out without splashing of the liquid. As shown in Figs. 12 to 14, in the squeezing process, the second buckle 9 hooks the front end of the sampling head 203, and the sample collector 200 realizes a lever movement with the contact part of the second buckle 9 and the sampling head 203 as a pivot until the wedge-shaped structure 202a of the connecting portion 202 of the sample collector squeezes and passes through the pair of hook tips 15, and remains under the pair of hook tips 15. The liquid sample within the sampling head 203 is sufficiently squeezed out (as shown in Fig. 15).

Preferably, the second buckle 9 is disposed in the storage chamber 6 of the test device. In order to further improve the buckling effect of the second buckle 9, the tail end of the second buckle 9 is provided with a bump 91 (as shown in Fig. 8), and the sampling head 203 of the sample collector is provided with a recess 206 (as shown in Fig. 1), so that when the sampling head 203 of the sample collector is placed into the storage chamber 6 of the test kit, the bump 91 of the second buckle is buckled in the recess 206 of the sampling head of the sample collector, thereby hooking the top end of the sampling head 203.

As shown in Fig. 6, the buckle 10 is disposed behind the rear enclosure 52. The gap between the pair of clamping hooks 14 of the buckle 10 is a clamping slot 13, which is on the same axis as the groove 12 of the rear enclosure. After the sample collector 200 squeezes the sampling pad 204 on the sampling head completely against the bottom plate 7, the connecting portion 202 of the sample collector is buckled into the buckle 10 and is confined within the clamping slot 13, and the sampling pad 204 remains in a squeezed state after the operator releases the handle 201 of the sample collector. The pair of clamping hooks 14 is clamped in cooperation with the wedge-shaped structure 202a so that the wedge-shaped structure 202a is confined within the clamping slot 13 to hold the sample collector 200 on the test kit 300, and the sampling pad 204 is sufficiently squeezed to squeeze the sample out sufficiently.

Please refer to Figs. 1 and 2, the sampling head 203 of the sample collector placed in the storage chamber 6 takes the second buckle 9 as a pivot to press down the sampling pad 204, so that the sampling pad 204 is squeezed by the bottom plate 7 to squeeze out the liquid sample within the sampling pad 204. As in Figs. 1 and 2, and Figs. 5 to 9, the second buckle 9 is provided with a bump 91 at the upper end (as shown in Fig. 8), and the sample collector is provided with a recess 206 at the top end (as shown in Fig. 5). When the sampling head 203 of the sample collector is inserted into the storage chamber 6, the top end of the sampling head 203 is placed under the second buckle 9, the bump 91 of the second buckle 9 is buckled in the recess 206 of the sample collector (as shown in Fig. 1), and then takes the buckled point as the pivot to rotate to press down the handle 201 of the sample collector, so that the sampling pad 204 is squeezed by the bottom plate 7. The manner of forming pivot fit between the second buckle 9 and the sampling head 203 may also be in other manner, but not limited to, those listed below, for example, the upper end of the second buckle 9 is provided as a recess and the top end of the sample collector includes a bump in cooperation with the recess. Alternatively, the second buckle 9 is provided with an aperture, the sampling head 203 is provided with a bugle at the top end, and the bugle can be inserted into the aperture to form a pressing pivot. These altered design solutions are not shown in the figures.

It's better that the second buckle 9 has certain elasticity. The second buckle 9 is disposed at a position within the storage chamber 6 and close to the front end of the storage chamber. For example, the second buckle 9 may be disposed on the inner wall of the front enclosure 51. For another example as shown in Fig. 7, the second buckle 9 is disposed on the bottom plate region between the sample addition hole 3 and the front enclosure 51, and a spacing space 11 is left between the second buckle 9 and the front enclosure 51 (as shown in Fig. 7). The spacing space 11 reserves a retraction space for the second buckle 9. It is specifically embodied in that when the sampling head 203 is inserted into the storage chamber 6, the front end of the sampling head presses against a clamping arm 92 of the second buckle 9 (as shown in Fig. 8), and the clamping arm 92 after being pressed undergoes elastic tilt towards the spacing space 11 until the bump 91 of the second buckle is buckled in the recess 206 of the sampling head, and the clamping arm 92 is elastically restored to its original position.

In the example where the spacing space 11 is provided between the second buckle 9 and the front enclosure 51, the spacing space 11 may also play a role of cushioning. In the solution that the second buckle 9 is disposed on the inner wall of the front enclosure, the top end of the sampling head 203 inserted into the storage chamber 6 is very close to the front enclosure 51, so that if the amount of the liquid sample squeezed out of the sampling pad 204 is larger than the gap between the top end of the sampling head 203 and the front enclosure 51, and this part of liquid sample has no time to flow into the test kit, the liquid sample located within the gap between the top end of the sampling head 203 and the front enclosure 51 might be squeezed out of the enclosure 5, thus the provided spacing space 11 increases the temporary storage space for the liquid sample, which is equivalent to a buffer region.

As shown in Figs. 1, 2, and 6, the rear enclosure 52 is also provided with a groove 12 for receiving the connecting portion 202 of the sample collector. When the sample collector 200 is pressed down by taking the second buckle 9 as a pivot, the connecting portion 202 of the collector can be embedded in the groove 12 of the rear enclosure, so that the sampling pad 204 of the sample collector 200 can be completely squeezed on the bottom plate 7 in a flat manner, thus sufficiently squeezing every position on the sampling pad 204.

### Example 1 Preparation of a portable SARS-CoV-2 test device

The preparation of a portable SARS-CoV-2 test device is taken as an example.

As shown in Fig. 4, the SARS-CoV-2 test strip 100 includes a bottom card 101, and a sample pad 102, a label binding pad 103, a test pad 104 and an absorbent pad 105 are adhered to the bottom card 101 in a manner of being superposed on each other in sequence from upstream to downstream. The label binding pad 103 is coated with an anti-SARS-CoV-2 antibody-latex label, the test line (T line) of the test pad is coated with an anti-SARS-CoV-2 antibody, and the control line (C line) is coated with goat-anti-mouse IgG.

The prepared SARS-CoV-2 test strip is installed in the test kit 300 such that the sample addition hole 3 of the upper cover 1 of the test kit is located above the sample pad 102 of the test strip and the observation window 4 is located above the test pad 104 of the test strip.

The sample collector 200 is encapsulated in a sealing bag to form a complete test device with the prepared test kit. The sampling pad 204 of the sample collector 200 absorbs water and swells upon contact with saliva.

### Example 2 Use of the test device

The use of the portable SARS-CoV-2 test device is taken as an example.

The sample collector 200 is removed from the sealing bag, and the sample collector is put into the mouth of a tester to aspirate saliva to a specified sample volume. If an indicator test strip is available, one can determine whether a sufficient volume has been collected according to the color change of the indicator test strip. If no indicator test strip is available, one can determine whether a sufficient volume has been collected according to the swelling degree of the sampling pad.

The sampling head 203 with sufficient volume collected is inserted into the storage chamber 6 of the test kit, the second buckle 9 within the storage chamber is buckled with the recess 206 at the front end of the sampling head, the sample collector 200 is pressed down by taking the second buckle 9 as the pivot, the connecting portion 202 of the collector is embedded into the groove 12 of the rear enclosure, and when the sample pad 204 of the sampling head is completely squeezed on the bottom plate 7 in a flat manner, the connecting portion 202 is buckled into the buckle 10 and is confined within the clamping slot 13, at which point the operator can release the collector and the sampling pad 204 can be held in the squeezed state all the time.

The liquid sample squeezed out of the sampling pad of the collector reaches the sample pad 102 of the test strip through the sample addition hole 3. Then, the liquid sample reaches the absorbent pad 105 via the sample pad 102, the label binding pad 103, and the test pad 104. If the test pad only shows a color at the position of the C line, it indicates that the sample is negative, if a color is shown at positions of both the C line and the T line, it indicates that the sample is positive and further nucleic acid analysis is required, and if no color is shown at the position of the C line, it indicates that this test is invalid.

### Example 3 Portable drug test device and use thereof

The drug test strip 100 is also referred to as a drug of abuse test strip, and "drug of abuse" (DOA) is a drug that is used for non-medical purposes (usually for psychedelic effects). Abuse of such drug can lead to physical and mental hurts as well as (in some cases) dependency, addiction, and even death. Instances of DOA include cocaine, amphetamines (e.g., black beauties, white bennies, amphetamine tablets, dextroamphetamines, dexies, beans), methamphetamines (crank, methamphetamine, crystal, speed), barbiturates (Valium^{®}, Roche Pharmaceuticals, Nutley, New Jersey), sedatives (i.e., sleeping pills), lysergic acid diethylamide (LSD), tranquilizers (downers, goofballs, barbs, blue devils, yellow jackets, ludes), tricyclic antidepressants (TCAs, e.g., promethazine, amitriptyline, and doxepin), phencyclidine (PCP), tetrahydrocannabinol (THC, pot, dope, hash, weed, etc.), and opiates (e.g., morphine, opium, cocaine, heroin, oxycodone).

A portable amphetamine (urine) colloidal gold test device is taken as an example in this example.

The portable amphetamine (urine) colloidal gold test device includes a test strip, a test kit and a sample collector. The test strip is a portable amphetamine (urine) colloidal gold test strip, including a bottom card, and a sample pad, a label binding pad, a test pad and an absorbent pad are adhered to the bottom card in a manner of being superposed on each other in sequence from upstream to downstream. The principle of binding amphetamine couplers and amphetamine that may be contained in the urine through competition of monoclonal antibodies. The label binding pad contains an anti-amphetamine monoclonal antibody (colloidal gold antibody) labeled with colloidal gold, and the T line on the test pad contains an amphetamine coupler.

During the test, the sample collector enters the urine of the tester and the urine sample on the collector is then squeezed into the test kit, where the urine sample is then chromatographed upwards on the test strip by capillary effect. If the concentration of amphetamine in the urine sample is less than 1000ng/ml, the colloidal gold antibody cannot bind to all of the amphetamine. Thus, the colloidal gold antibody will be bound by the amphetamine coupler immobilized on the test pad in the chromatographic process and a purple-red stripe will appear on the T line. If the concentration of amphetamine in the urine sample is higher than 1000ng/ml, the colloidal gold antibody binds to all of the amphetamine, and thus no purple-red stripe appears in the T-line region due to no bonding to the amphetamine coupler because of a competing reaction.

The negative urine sample will show a purple-red stripe on the T line due to the lack of antibody-antigen competing reactions in the testing process. A purple-red stripe will appear at the control line C no matter whether amphetamine is present in the urine sample or not. The purple-red stripe shown in the control region (C) is the standard for determining whether a sufficient urine sample is available and whether the chromatographic process is normal, and also serves as an internal controlled standard for the reagent.

In the example where one test device is used for testing multiple drugs of abuse, a plurality of test strip storage slots are formed in the lower plate of the test kit for placing test strips for different test items.

Example 4 Portable immunofluorescence analysis (FIA) test device and use thereof The test strip in this example takes time-resolved immunofluorescence testing based on the phosphorescence luminance technology as an example.

The test strip is used for testing IgG antibodies generated after infection by a pathogenic microorganism, with the test mode being an indirect method mode. The preparation method of the test strip includes: labeling an anti-human immunoglobulin IgG antibody with a phosphorescent material and immobilizing the labeled antibody on the label bonding pad; immobilizing the pathogenic microbial antigen to be analyzed on the test line of the test pad; and immobilizing the IgG on the control line of the test pad.

During testing, the liquid sample on the collector is squeezed into the test kit, then the collector is pulled out, the test kit containing the test strip described in this example is inserted into a fluorescence analyzer, and the test result is analyzed through the value of the intensity of phosphorescent signals of the T line and C line of the test strip. When both the test line and the control line generate a phosphorescent signal at the same time, it is a positive result, indicating that the sample contains the target substance to be tested; and when the test line does not generate a phosphorescent signal but the control line generates a phosphorescent signal, it is a negative result, indicating that the sample does not contain the target substance to be tested.

The test device described in the present invention can be used in testing fields such as disease diagnosis, driving under the influence (DUI) test, drug taking situation, and hormone analysis, which meets the requirements of rapid sampling and point-of-care testing. The types of samples that can be detected by the test device described in the present invention can be urine, saliva, blood, sample lysate, etc.

## Claims

1. A test device, comprising a sample collector and a test kit, the test kit is used to store a test strip, wherein the sample collector comprises a sampling head, a handle and a connecting portion between the sampling head and the handle, and the test kit comprises a sample addition hole and a buckle; when the sample collector is placed into the test kit, the connecting portion of the sample collector is fastened to the buckle of the test kit, and the connecting portion of the sample collector comprises a wedge-shaped structure wide at the top and narrow at the bottom, the wedge-shaped structure being located at the point where the sample collector is in fastening fit with the buckle.

2. The test device according to claim 1, wherein the buckle of the test kit comprises a pair of inverted clamping hooks, the hook tips of which face inward towards each other; after the sampling head of the sample collector is placed into the storage chamber of the test kit, the wedge-shaped structure of the sample collector squeezes the buckle downward, causing the pair of inverted clamping hooks to open outward; and after the wedge-shaped structure passes through the hook tips, the clamping hooks spring back and hook the wedge-shaped structure.

3. The test device according to claim 2, wherein the wedge-shaped structure comprises at least one beveled or cambered surface which guides the clamping hooks to open outward when the wedge-shaped structure squeezes the buckle downward.

4. The test device according to claim 1, wherein the shape of at least the lower part of the cross section of the wedge-shaped structure comprises: an inverted trapezoid, an inverted triangle, a combination of an inverted trapezoid and a rectangle, a semicircle, or a shape approximate to a semicircle.

5. The test device according to claim 1, wherein the cross section of the wedge-shaped structure is in the shape of inverted " 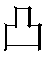 ".

6. The test device according to claim 2, wherein the width of the bottom of the wedge-shaped structure is less than the distance between the two hook tips of the clamping hook.

7. The test device according to claim 6, wherein the width of the bottom of the wedge-shaped structure is less than one half of the width of the top thereof.

8. The test device according to claim 1, wherein the test kit further comprises a second buckle that catches the sampling head of the sample collector after the sampling head of the sample collector is placed into the storage chamber of the test device.

9. The test device according to claim 8, wherein the second buckle is disposed in the storage chamber of the test device.

10. The test device according to claim 9, wherein the tail end of the second buckle is provided with a bump, and the sampling head of the sample collector is provided with a recess, when the sampling head of the sample collector is placed into the storage chamber of the test kit, the bump of the second buckle is buckled in the recess of the sampling head of the sample collector.

11. A sample collector, comprising a sampling head, a handle and a connecting portion between the sampling head and the handle, wherein the connecting portion of the sample collector comprises a wedge-shaped structure wide at the top and narrow at the bottom.

12. The test device according to claim 11, wherein the wedge-shaped structure comprises at least one beveled or cambered surface.

13. The test device according to claim 11, wherein the shape of at least the lower part of the cross section of the wedge-shaped structure comprises: an inverted trapezoid, an inverted triangle, a combination of an inverted trapezoid and a rectangle, a semicircle, or a shape approximate to a semicircle.

14. The test device according to claim 11, wherein the cross section of the wedge-shaped structure is in the shape of inverted "1".
The test device according to claim 11, wherein the width of the bottom of the wedge-shaped structure is less than one half of the width of the top thereof.
